# EUROPEAN PATENT APPLICATION

(11) **EP 4 834 940 A1**
(43) Date of publication of application: **04.11.2026**
(21) Application number: 24911550.2
(22) Date of filing: 27.12.2024
(51) Int. Cl.: C07K 16/28, C07K 19/00, A61K 47/68, A61K 39/395

(54) **ANTI-CD276 NANOBODY, PREPARATION METHOD THEREFOR AND USE THEREOF**

(30) Priority: 28.12.2023 CN 202311838428
(71) Applicant: Persongen Biotherapeutics (Suzhou) Co., Ltd., Suzhou, Jiangsu 215123 (CN)
(72) Inventor: YANG, Lin, Suzhou, Jiangsu 215123 (CN); YOU, Fengtao, Suzhou, Jiangsu 215123 (CN)
(74) Representative: dompatent
(86) International application number: PCT/CN2024/143398
(87) International publication number: WO 2025/140634

(57) **Abstract**

Provided are an anti-CD276 nanobody, and a preparation method therefor and the use thereof. Specifically, further provided are a corresponding ani-CD276 antibody, a chimeric antigen receptor, a fusion protein, a recombinant protein, an antibody-drug conjugate, and an encoding nucleic acid, an expression vector, a host cell, etc. thereof. The anti-CD276 nanobody is high in affinity, simple in structure and easy to prepare, and the anti-CD276 nanobody-drug conjugate has a significant killing activity, thereby having application prospects in the fields such as tumor treatment and immunodetection.

## Description

### TECHNICAL FIELD

The present invention relates to the field of biomedicine, and more particularly to an anti-CD276 nanobody, and a preparation method therefor and the use thereof.

### BACKGROUND

CD276 molecule, also known as B7-H3 molecule, is a kind of type I transmembrane glycoprotein, belonging to the B7 immune costimulatory and co-inhibitory globulin family. The existing studies show that CD276 molecules have both immune activation and immunosuppressive effects. On one hand, the CD276 molecule can play an immunosuppressive effect by weakening the secretion of type I cytokine IFN-gamma, and many research results support that CD276 has a negative immunomodulatory effect in graft versus host disease, heart transplant rejection, airway inflammation and autoimmune encephalomyelitis model. On the other hand, CD276 is also found to exert a function of providing an immune co-stimulatory signal for T cells in autoimmune diseases *in vivo* and *in vitro*.

A large number of studies show that CD276 is overexpressed in various human tumors, comprising leukemia, gastric cancer, breast cancer, non-small cell lung cancer, ovarian cancer, osteosarcoma, neuroblastoma, pancreatic cancer, breast cancer and the like, but has only limited expression in normal tissue. At the same time, it is also reported to be expressed in important tumor microenvironment components such as tumor stem cells, tumor stromal cells and tumor neovessels. The overexpression of CD276 can generally reduce the infiltration of tumor-associated lymphocytes in tumors and accelerate disease progression, and is generally closely related to the poor prognosis of cancers such as pancreatic ductal cancer, prostate cancer, ovarian cancer, lung cancer and transparent cell renal cancer.

Therefore, there is currently a lack of anti-CD276 nanobody with high affinity, high specificity and high biological activity against CD276. Therefore, there is an urgent need in the art to develop anti-CD276 nanobody with high affinity, high specificity and high biological activity.

### SUMMARY OF THE INVENTION

The purpose of the present invention is to provide an anti-CD276 nanobody with high affinity, high specificity and high biological activity.

In the first aspect of the present invention, it provides an anti-CD276 nanobody, wherein the anti-CD276 nanobody has one or more complementarity-determining regions (CDRs) selected from the group consisting of:
(1) CDR1 as set forth in SEQ ID NO: 6, CDR2 as set forth in SEQ ID NO: 7, and CDR3 as set forth in SEQ ID NO: 8;
(2) CDR1 as set forth in SEQ ID NO: 9, CDR2 as set forth in SEQ ID NO: 10, and CDR3 as set forth in SEQ ID NO: 11;
(3) CDR1 as set forth in SEQ ID NO: 12, CDR2 as set forth in SEQ ID NO: 13, and CDR3 as set forth in SEQ ID NO: 14;
(4) CDR1 as set forth in SEQ ID NO: 15, CDR2 as set forth in SEQ ID NO: 16, and CDR3 as set forth in SEQ ID NO: 17;
(5) CDR1 as set forth in SEQ ID NO: 18, CDR2 as set forth in SEQ ID NO: 19, and CDR3 as set forth in SEQ ID NO: 20.

In another preferred embodiment, the CDR1, CDR2 and CDR3 are separated in sequence by framework regions FR1, FR2, FR3 and FR4 of a VHH chain, respectively.

In another preferred embodiment, the amino acid sequence of the VHH chain of the anti-CD276 nanobody is as shown in any one of SEQ ID NOs: 1-5.

In another preferred example, the VHH chain of the anti-CD276 nanobody comprises an amino acid sequence having a sequence similarity of at least 80%, preferably at least 90%, more preferably at least 95%, even more preferably at least 99% to any one of SEQ ID NOs: 1-5.

In another preferred embodiment, the amino acid sequence of the VHH chain of the anti-CD276 nanobody comprises one or more amino acid substitutions compared to any one of SEQ ID NOs: 1-5, preferably conservative amino acid substitutions.

In another preferred embodiment, any one of the aforementioned amino acid sequences further comprises a derivative sequence obtained by optionally adding, deleting, modifying and/or substituting at least one (e.g., 1-3, preferably 1-2, more preferably 1) amino acid and being capable of retaining the ability of specific binding to CD276.

In another preferred embodiment, the anti-CD276 nanobody compries humanized antibodies, animal-derived antibodies, and chimeric antibodies.

In the second aspect of the present invention, it provides an anti-CD276 multivalent antibody or antibody against multiple epitopes, wherein the multivalent antibody or antibody against multiple epitopes comprises at least one antibody element against a CD276 epitope, and the antibody element is the anti-CD276 nanobody described in the first aspect of the present invention.

In another preferred embodiment, the anti-CD276 multivalent antibody or antibody against multiple epitopes comprises one or more anti-CD276 nanobodies.

In another preferred embodiment, the anti-CD276 multivalent antibody comprises a monomer, a bivalent body (divalent antibody), a tetravalent body (tetravalent antibody), and/or a multivalent body (multivalent antibody).

In another preferred embodiment, the anti-CD276 multivalent antibody comprises one or more VHH chains having an amino acid sequence as shown in any one of SEQ ID NOs: 1-5.

In another preferred embodiment, the anti-CD276 multivalent antibody comprises two VHH chains having the amino acid sequences shown in SEQ ID NOs: 1-5.

In another preferred embodiment, the anti-CD276 nanobody comprises humanized antibodies, animal-derived antibodies, and chimeric antibodies.

In the third aspect of the present invention, it provides a recombinant protein, wherein the recombinant protein comprises:
(i) the anti-CD276 nanobody of the first aspect of the present invention, or the anti-CD276 multivalent antibody or antibody against multiple epitopes of the second aspect of the present invention; and
(ii) an optional tag sequence that facilitates expression and/or purification.

In another preferred embodiment, the tag comprises a FLAG tag, an Fc tag, an HA tag and His tag.

In another preferred embodiment, the tag is an His tag.

In another preferred embodiment, the recombinant protein specifically binds to CD276.

In the fourth aspect of the present invention, it provides an antibody-drug conjugate, wherein the antibody-drug conjugate comprises:
(a) the nanobody of the first aspect of the present invention, the multivalent antibody or antibody against multiple epitopes of the second aspect of the present invention, or the recombinant protein of the third aspect of the present invention; and
(b) a conjugated moiety conjugated to the antibody moiety, wherein the conjugated moiety is selected from the group consisting of: detectable labels, drugs, toxins, cytokines, radionuclides, enzymes, and combinations thereof.

In another preferred embodiment, the antibody moiety is conjugated to the conjugated moiety via chemical bonds or linkers.

In the fifth aspect of the present invention, it provides a chimeric antigen receptor (CAR), wherein the CAR comprises an extracellular domain that comprises the anti-CD276 nanobody of the first aspect of the present invention, or the anti-CD276 multivalent antibody or antibody against multiple epitopes of the second aspect of the present invention.

In another preferred embodiment, the extracellular domain further comprises a signal peptide.

In another preferred embodiment, the extracellular domain further comprises other heterologous proteins.

In another preferred embodiment, the CAR has a structure represented by Formula (I):

L-Nb-H-TM-C-CD3ζ (I)

wherein,
the "-" is a linker peptide or a peptide bond;
L is absent or a signal peptide sequence;
Nb is an antigen-binding domain that specifically binds to CD276;
H is absent or a hinge region;
TM is a transmembrane domain;
C is a costimulatory signaling domain;
CD3ζ is a cytoplasmic signaling sequence derived from CD3ζ (comprising the wild-type form, or mutants/modified forms thereof).

In another preferred embodiment, the L is a signal peptide of proteins selected from the group consisting of: CD8, 4-1BB, GM-CSF, CD3, CD8a, and combinations thereof.

In another preferred embodiment, the sequence of the Nb is as shown in any one of SEQ ID NOs: 1-5.

In another preferred embodiment, the H is a hinge region selected from the group consisting of proteins: CD8, CD28, CD137, IgG, and combinations thereof.

In another preferred embodiment, the C is a costimulatory domain of proteins selected from the group consisting of: OX40, CD2, CD7, CD27, CD28, CD30, CD40, CD70, CD134, 4-1BB (CD137), PD-1, Dap10, LIGHT, NKG2C, B7-H3, ICAM-1, LFA-1 (CD11a/CD18), ICOS (CD278), NKG2D, GITR, OX40L, 2B4, TLR, and mutants/modified forms thereof, and combinations thereof.

In the sixth aspect of the present invention, it provides a polynucleotide, wherein the polynucleotide encodes a protein selected from the group consisting of: the nanobody of the first aspect of the present invention, the multivalent antibody or antibody against multiple epitopes of the second aspect of the present invention, or the recombinant protein of the third aspect of the present invention, and the CAR of the fifth aspect of the present invention.

In the seventh aspect of the present invention, it provides an expression vector, wherein the expression vector comprises the polynucleotide of the sixth aspect of the present invention.

In another preferred embodiment, the expression vector is selected from the group consisting of: DNA, RNA, viral vectors, plasmids, transposons, other gene transfer systems, and combinations thereof.

In another preferred embodiment, the expression vector is a lentiviral vector.

In the eighth aspect of the present invention, it provides a host cell, wherein the host cell comprises the expression vector of the seventh aspect of the present invention, or has the polynucleotide of the sixth aspect of the present invention integrated into its genome, or expresses the nanobody of the first aspect of the present invention, the multivalent antibody or antibody against multiple epitopes of the second aspect of the present invention, the recombinant protein of the third aspect of the present invention, or the CAR of the fifth aspect of the present invention.

In another preferred embodiment, the cell is an isolated cell, and/or the cell is a genetically engineered cell.

In another preferred embodiment, the cells are mammalian cells.

In another preferred embodiment, the cells are T cells.

In another preferred embodiment, the host cell is an engineered immune cell.

In another preferred embodiment, the engineered immune cell is selected from the group consisting of:
(i) chimeric antigen receptor αβT cells (CAR-T cells);
(ii) chimeric antigen receptor γδT cells (CAR-T cells);
(iii) chimeric antigen receptor NKT cells (CAR-NKT cells);
(iv) chimeric antigen receptor NK cells (CAR-NK cells);
(v) chimeric antigen receptor macrophages.

In another preferred embodiment, the engineered immune cell comprises autologous or allogeneic αβT cells, γδT cells, NKT cells, NK cells, and combinations thereof.

In another preferred embodiment, the engineered immune cell is a CAR-T cell.

In the ninth aspect of the present invention, it provides a preparation, wherein the preparation comprises the nanobody of the first aspect of the present invention, the multivalent antibody or antibody against multiple epitopes of the second aspect of the present invention, the recombinant protein of the third aspect of the present invention, the antibody-drug conjugate of the fourth aspect of the present invention, the CAR of the fifth aspect of the present invention, or the vector of the seventh aspect of the present invention, or an immune cell expressing the CAR of the fifth aspect of the present invention, and a pharmaceutically acceptable carrier, diluent or excipient.

In the tenth aspect of the present invention, it provides a pharmaceutical composition, comprising:
(i) the nanobody of the first aspect of the present invention, the multivalent antibody or antibody against multiple epitopes of the second aspect of the present invention, the recombinant protein of the third aspect of the present invention, the antibody-drug conjugate of the fourth aspect of the present invention, or the host cell of the eighth aspect of the invention; and
(ii) a pharmaceutically acceptable carrier.

In another preferred embodiment, the pharmaceutical composition further comprises other drugs for treating diseases.

In another preferred embodiment, the pharmaceutical composition is used to prepare a drug for preventing and/or treating diseases or conditions associated with CD276.

In another preferred embodiment, the diseases or conditions associated with CD276 is selected from the group consisting of: leukemia, gastric cancer, breast cancer, non-small cell lung cancer, ovarian cancer, osteosarcoma, neuroblastoma, pancreatic cancer, breast cancer, and combinations thereof.

In the eleventh aspect of the present invention, it provides a kit comprising the polynucleotide of the sixth aspect of the present invention, the CAR of the fifth aspect of the present invention, or the vector of the seventh aspect of the present invention.

In the twelfth aspect of the present invention, it provides a use of the nanobody of the first aspect of the present invention, the multivalent antibody or antibody against multiple epitopes of the second aspect of the present invention, the recombinant protein of the third aspect of the present invention, the antibody-drug conjugate of the fourth aspect of the present invention, or the immune cell expressing the CAR of the fifth aspect of the present invention in the preparation of a medicament for preventing and/or treating cancers or diseases associated with CD276.

In another preferred embodiment, the cancers or diseases associated with CD276 is selected from the group consisting of: leukemia, gastric cancer, breast cancer, non-small cell lung cancer, ovarian cancer, osteosarcoma, neuroblastoma, pancreatic cancer, breast cancer, and combinations thereof.

In the thirteenth aspect of the present invention, it provides a method for treating a disease, which comprises administering to a subject in need of treatment: the nanobody of the first aspect of the present invention, the multivalent antibody or antibody against multiple epitopes of the second aspect of the present invention, the recombinant protein of the third aspect of the present invention, the antibody-drug conjugate of the fourth aspect of the present invention, or the immune cell expressing the CAR of the fifth aspect of the present invention.

In another preferred embodiment, the disease is a tumor that is positive for CD276 expression.

In another preferred embodiment, the subject comprises mammals, such as humans.

In another preferred embodiment, the tumor is selected from the group consisting of: leukemia, gastric cancer, breast cancer, non-small cell lung cancer, ovarian cancer, osteosarcoma, neuroblastoma, pancreatic cancer, breast cancer, and combinations thereof.

It should be understood that, within the scope of the present invention, the above-described technical features of the present invention and the technical features specifically described below (such as in the examples) can be combined with each other to form new or preferred technical solutions. Due to space limitations, they will not be described in detail here.

### DESCRIPTION OF THE DRAWINGS

Figure 1 shows the result of agarose gel electrophoresis of VHH fragment PCR products.
Figure 2 shows the result of the diversity analysis of the phage display library.
Figure 3 shows the result of phage ELISA detection of single clones from the enriched products.
Figure 4 shows the result of flow cytometry detection of binding of candidate antibodies to the target protein.
Figure 5 shows the result of the internalization activity assay of candidate antibodies.
Figure 6 shows the result of the cytotoxicity activity assay of the candidate antibody ADC.
Figure 7 shows the result of the antibody affinity assay.

### DETAILED DESCRIPTION

Through extensive and in-depth research, the present inventors unexpectedly obtained, through extensive screening, for the first time, anti-CD276 nanoantibodies with high affinity and high specificity. Specifically, the present invention used CD276 recombinant protein to immunize camels, thereby obtaining a high-quality immune library. Phage display technology was used to screen the immune library, resulting in a class of specific nanoantibodies against CD276. Based on the nanoantibodies, the present invention also provides antibody-drug conjugates with significant cytotoxic effects. The anti-CD276 nanoantibodies of the present invention have high affinity, simple structure, and are easy to prepare, with broad application prospects in fields such as tumor treatment and immunoassays. On this basis, the present invention has been completed.

### TERMS

To facilitate understanding of the present disclosure, certain terms are first defined. As used herein, unless otherwise explicitly specified in the text, each of the following terms shall have the meaning given below. Other definitions are set forth throughout the application.

The term "about" may refer to a value or composition within an acceptable error range of a specific value or composition determined by those of ordinary skill in the art, which will depend in part on how the value or composition is measured or determined. For example, as used herein, the expression "about 100" comprises all values between 99 and 101 (e.g., 99.1, 99.2, 99.3, 99.4, etc.).

As used herein, the terms "comprising" or "including" may be open, semi-closed, or closed. In other words, the terms also comprise "consisting essentially of" or "consisting of."

Sequence identity is determined by comparing two aligned sequences along a predetermined comparison window (which can be 50%, 60%, 70%, 80%, 90%, 95% or 100% of the length of the reference nucleotide sequence or protein) and determining the number of positions at which identical residues occur. Typically, this is expressed as a percentage. The measurement of sequence identity of nucleotide sequences is a method well known to those skilled in the art.

### Antibody

As used herein, the term "antibody" refers to immunoglobulins, which are tetrapeptide chains composed of two identical heavy chains and two identical light chains connected by interchain disulfide bonds.

As used herein, the terms "single-domain antibody (VHH)" and "nanobody" have the same meaning and can be used interchangeably, referring to a single-domain antibody (VHH) composed of only one heavy chain variable region cloned from the variable region of an antibody heavy chain, which is the smallest antigen-binding fragment with complete function. Usually, after obtaining an antibody that naturally lacks the light chain and heavy chain constant region 1 (CH1), the variable region of the antibody heavy chain is cloned to construct a single-domain antibody (VHH) composed of only one heavy chain variable region.

The term "antigen-binding fragment of an antibody" (or simply "antibody fragment") refers to one or more fragments of an antibody that retain the ability to specifically bind to an antigen (e.g., CD276). It has been shown that fragments of a full-length antibody can perform the antigen-binding function of the antibody. Examples of binding fragments included within the term "antigen-binding fragment of an antibody" comprise:
(i) Fab fragments, monovalent fragments composed of VL, VH, CL, and CH1 domains;
(ii) F(ab')₂ fragments, bivalent fragments comprising two Fab fragments linked by a disulfide bridge at the hinge region;
(iii) Fd fragments, composed of VH and CH1 domains;
(iv) Fv fragments, composed of the VH and VL domains of a single arm of an antibody.

Fv antibodies comprise the heavy chain variable region and light chain variable region of an antibody, but lack constant regions, and are the smallest antibody fragments with all antigen-binding sites. Generally, Fv antibodies also comprise a polypeptide linker between the VH and VL domains and are capable of forming the structure required for antigen binding.

The term "CDR" refers to one of the six hypervariable regions within the variable domains of an antibody that primarily contribute to antigen binding. One of the most commonly used definitions for these six CDRs is provided by Kabat E.A. et al., (1991) Sequences of Proteins of Immunological Interest, NIH Publication No. 91-3242.

The term "epitope" or "antigenic determinant" refers to a site on an antigen to which an immunoglobulin or antibody specifically binds (e.g., a specific site on the CD276 molecule). Epitopes generally comprise at least 3, 4, 5, 6, 7, 8, 9, 10, 11, 12, 13, 14, or 15 consecutive or non-consecutive amino acids in a unique spatial conformation.

The terms "specific binding," "selective binding," "selectively binds," and "specifically binds" refer to the binding of an antibody to a predetermined epitope on an antigen. Typically, the antibody binds with an affinity (KD) of approximately less than 10⁻⁷ M, for example, approximately less than 10⁻⁸ M, 10⁻⁹ M, or 10⁻¹⁰ M or lower.

As used herein, the term "antigenic determinant" refers to a discontinuous, three-dimensional spatial site on an antigen that is recognized by the antibody or antigen-binding fragment of the present invention.

The present invention comprise not only intact antibodies but also immunologically active antibody fragments or fusion proteins formed by antibodies with other sequences. Therefore, the present invention also comprises fragments, derivatives, and analogs of the antibodies.

In the present invention, the antibodies of the invention also comprise conservative variants thereof, which refer to polypeptides in which, compared to the amino acid sequence of the antibody of the invention, up to 10, preferably up to 8, more preferably up to 5, and most preferably up to 3 amino acids are substituted with amino acids of similar or analogous properties. These conservative variant polypeptides are preferably produced by amino acid substitutions according to Table A below.

**Table A**

| Original Residue | Representative Substitution | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-CD276 Nanobody

As used herein, the term "CD276" generally refers to natural or recombinant human CD276, as well as non-human homologs of human CD276.

As used herein, the terms "nanobody of the present invention," "single-domain antibody of the present invention," and "anti-CD276 nanobody of the present invention" are used interchangeably and all refer to the nanobodies of the present invention that possess high specificity and high affinity for CD276.

Compared to traditional monoclonal antibodies, the nanobodies of the present invention have small molecular mass, strong penetrating ability, high sensitivity, high specificity, and good stability. Upon entering the body, they can efficiently penetrate cells to rapidly capture antigens for therapeutic purposes.

In the present invention, the anti-CD276 nanobody comprises a monomer, a bivalent body (divalent antibody), a tetravalent body (tetravalent antibody), and/or a multivalent body (multivalent antibody).

In a preferred embodiment of the present invention, the amino acid sequence of the VHH chain of the anti-CD276 nanobody is selected from one or more of SEQ ID NOs: 1-5. The preferred nanobody designations, VHH sequence identifiers, and corresponding CDR region sequence identifiers of the present invention are shown in Table 1:

**Table 1**

| **Antibody Name** | **CDR1** | **CDR2** | **CDR3** | **The amino acid sequence identifier of the VHH chain** |
|---|---|---|---|---|
| 1-B1 | 6 | 7 | 8 | 1 |
| 8-E5 | 9 | 10 | 11 | 2 |
| 1-E3 | 12 | 13 | 14 | 3 |
| 1-A2 | 15 | 16 | 17 | 4 |
| 4-B11 | 18 | 19 | 20 | 5 |

| | | | | |
|---|---|---|---|---|
| Note: The numerical values in the table represent sequence identifiers, i.e., "1" indicates "SEQ ID NO: 1." | | | | |

### Antibody-Drug Conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, comprising imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a cleavable or non-cleavable linker. Typically, cleavable linkers are easily degraded in an intracellular environment, for example, the linker is degraded at the target site, thereby releasing the drug from the antibody. Suitable cleavable linkers include, for example, enzyme-cleavable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable cleavable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-cleavable linker typically releases a drug under conditions that the antibody is hydrolyzed by protease.

Prior to linkage to the antibody, a linker has a active reactive group capable of reacting with certain amino acid residues, and the linkage is a chieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH₃COO⁻, Cl⁻ or NO₃⁻; and polymethylene dimethyl sulfide thiosulfonate. The linker may include, for example, a maleimide linked to an antibody via thiosuccimide.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive active group before being linked to an antibody.

Useful drugs icomprise, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc.In the present invention, a drug-linker can be used to form an ADC in a simple step. In other embodiments, a bifunctional linker compound can be used to form an ADC in a two-step or multi-step process. For example, a cysteine residue is reacted with the reactive active moiety of a linker in a first step, and then the functional group on the linker is reacted with a drug in the subsequent step, so as to form an ADC.

In general, the functional group on a linker is selected so that it can specifically react with the suitable reactive active group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups comprise, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows: 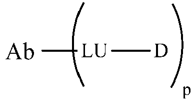 wherein,
Ab is a nanobody of the present invention;
LU is a linker;
D is a drug;
and the subscript p is a value selected from 1 to 8.

### Pharmaceutical composition

The present invention further provides a composition. In the preferred examples, the composition is a pharmaceutical composition comprising the antibody, or an active fragment, a fusion protein or an ADC thereof, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated. The formulated pharmaceutical composition may be administered by conventional routes, including (but not limited to): intratumoral, intraperitoneal, intravenous, or topical administration.

The antibody of the present invention may also be intracellularly expressed from nucleotide sequences for application in cell therapy, such as in chimeric antigen receptor T-cell immunotherapy (CAR-T), among others.

The pharmaceutical composition according to the present invention can be directly used for binding to a CD276 protein molecule, and therefore is useful for the prevention and treatment of CD276-related diseases. In addition, other therapeutic agents can be used simultaneously,

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 1 µg per kilogram of body weight to about 5 mg per kilogram of body weight daily. In addition, the polypeptide according to the present invention may also be used in combination with an additional therapeutic agent.

When a pharmaceutical composition is used, a safe and effective amount of an immunoconjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, a specific amount should also depend on the factors such as administration route and physical conditions of a patient, which fall into the skills of skilled physicians.

### The main advantages of the present invention comprise:

(1) The anti-CD276 nanobody of the present invention possesses high specificity and affinity.
(2) The anti-CD276 nanobody of the present invention has a small molecular mass, strong tissue penetration, and high sensitivity.
(3) The anti-CD276 nanobody of the present invention can be used to prepare antibody-drug conjugates with significant cytotoxic effects.
(3) The anti-CD276 nanobody of the present invention has a simple structure, is easy to prepare, and holds application prospects in fields such as tumor therapy and immunoassay.

The present invention will be further described below in conjunction with specific examples. It should be understood that these examples are intended to illustrate the present invention only and are not intended to limit the scope of the present invention. The experimental methods in the following examples, for which specific conditions are not specified, are generally performed under conventional conditions, such as those described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

### Example 1: Screening of Anti-CD276 Single-Domain Antibodies

### 1.1 Alpaca Immunization

An alpaca was immunized with recombinant CD276 protein (Q5ZPR3, Leu29-Ala466), with an immunization interval of 21 days. 10 days after the final immunization, 5 mL of peripheral blood was collected, and the serum was separated for evaluation of the immunization effect by ELISA.

### 1.2 Detection of Immune Titers

Peripheral blood (5 mL) was collected. The centrifuge tube containing the blood sample was placed in a 37 °C incubator for 1 hour; the blood sample was then transferred to 4 °C and kept overnight. The centrifuge tube containing the blood sample was centrifuged at 5000 rpm for 20 min. The upper serum layer was separated and transferred to a new sterile centrifuge tube, and the immune serum was collected. The target recombinant protein was diluted to a final concentration of 1 µg/mL using sterile CBS (carbonate buffer). A new 96-well ELISA plate was taken, and 100 µL/well of the diluted protein was added and incubated overnight at 4 °C for coating. The antigen coating solution was discarded, and the plate was washed 5 times with PBST (containing 0.05% Tween 20). Blocking was performed by adding 200 µL/well of 3% MPBS and incubating at 37 °C for 2 hours. After the blocking buffer was removed, the plate was washed 5 times with PBST. Serially diluted serum samples (100 µL/well) were added and incubated at room temperature for 1 hour, with PBS used as the control in the control wells. The liquid in the wells was discarded, and the plate was washed 5 times with PBST. HRP-conjugated anti-Lama IgG (H+L) antibody (diluted 1:50,000) was added at 100 µL/well and incubated at room temperature for 1 hour. After the liquid was discarded, the plate was washed 5 times with PBST. TMB substrate solution (100 µL/well) was added and incubated at room temperature in the dark for 10-15 minutes. Stop solution (50 µL/well) was then added. The OD450 value of each well was read using a microplate reader.

The results are shown in Table 2. Based on the ELISA results of the immune titers, the CD276 immune titer was found to be relatively high; thus, a booster immunization was performed, and 10 days later, 100 mL of peripheral blood was collected for the construction of a phage display library.

**Table 2. Detection results of immune titers**

| Serially diluted serum | CD450 | |
|---|---|---|
| 1:1K | 2.522 | 2.452 |
| 1:2K | 2.018 | 2.136 |
| 1:4K | 1.837 | 1.824 |
| 1:8K | 0.981 | 0.918 |
| 1:16K | 0.414 | 0.428 |
| 1:32K | 0.283 | 0.265 |
| 1:64K | 0.241 | 0.22 |
| PBS | 0.113 | 0.124 |

### 1.3 PBMC Isolation and Cloning of VHH Antibody Fragments

1. Peripheral blood (100 mL) was collected, and PBMCs were isolated using lymphocyte separation medium.
2. RNA was extracted, and reverse transcription was performed using the PrimeScript^{™} II 1st Strand cDNA Synthesis Kit to prepare cDNA. In a 200 µL PCR tube, the reaction mixture Mix1 was prepared as shown in Table 3:

**Table 3. Preparation system of reaction mixture Mix1**

| Reagent | Amount |
|---|---|
| Oligo dT Primer (50 µM) | 8µL |
| dNTP Mixture (10 mM each) | 8µL |
| Total RNA sample | 20µg |
| RNase-Free water | Bring to 80µL |

After incubation at 65 °C for 5 min, the mixture was rapidly cooled on ice. The following reaction solution was prepared in the above PCR tube as shown in Table 4:

**Table 4. PCR reaction solution**

| Reagent | Amount |
|---|---|
| Denatured reaction solution from above | 80µL |
| 5×PrimeScript II Buffer | 32µL |
| RNase Inhibitor (40 M/µL) | 4µL |
| PrimeScript II RTase (200 M/µL) | 8µL |
| RNase-Free water | 36µL |

After mixing by pipetting, the solution was aliquoted into 80 µL/tube and placed in a PCR instrument for incubation at 42 °C for 1 hour, followed by heat inactivation at 70 °C for 15 minutes. Finally, the cDNA samples were placed on ice or stored at -20 °C for long-term preservation.

### 3. Amplification of VHH fragments:

(1) The first-round PCR reaction system (50 µL/tube) was prepared as shown in Table 5:

**Table 5 First-round PCR reaction system**

| Component | Amount |
|---|---|
| Forward primer (5 µM) | 2µL |
| Reverse primer (10 µM) | 1µL |
| NuHi Power mix (2×) | 25µL |
| cDNA template | 2µL |
| Sterile water | 20µL |

After the PCR reaction system was prepared, the PCR instrument was programmed according to the settings shown in Table 6:

**Table 6 PCR program settings**

| Program | Temperature | Time | Cycles |
|---|---|---|---|
| Initial denaturation | 95°C | 10min | |
| Denaturation | 95°C | 15s | 25X |
| Annealing | 55°C | 30s | |
| Extension | 68°C | 1min | |
| Final extension | 68°C | 10min | |

(2) Agarose gel electrophoresis analysis of PCR products: The PCR products were analyzed by electrophoresis using 1% agarose gel, and fragments of approximately 750 bp in size were separated. The PCR products were recovered using a gel extraction kit, and the concentration was measured with a NanoDrop.
(3) The second-round PCR reaction system (50 µL/tube) was prepared as shown in Table 7:

**Table 7 Second-round PCR reaction system**

| Component | Amount |
|---|---|
| 2^{nd} F primer | 2µL |
| 2^{nd} R primer | 2µL |
| NuHi Power mix (2×) | 25µL |
| First-round PCR recovered product | 200ng |
| Sterile water | Bring to 50µL |

After the PCR reaction system was prepared, the PCR instrument was programmed according to the settings shown in Table 8:

**Table 8 Second-round PCR program settings**

| Program | Temperature | Time | Cycles |
|---|---|---|---|
| Initial denaturation | 95°C | 10min | |
| Denaturation | 95°C | 15s | |
| Annealing | 55°C | 30s | 18X |
| Extension | 68°C | 1min | |
| Final extension | 68°C | 10min | |

(4) Agarose gel electrophoresis analysis of the second-round PCR products: The PCR products were analyzed by electrophoresis using 1% agarose gel, and VHH fragments of approximately 400 bp in size were separated. The VHH PCR products were recovered using a gel extraction kit, and the concentration was measured with a NanoDrop.

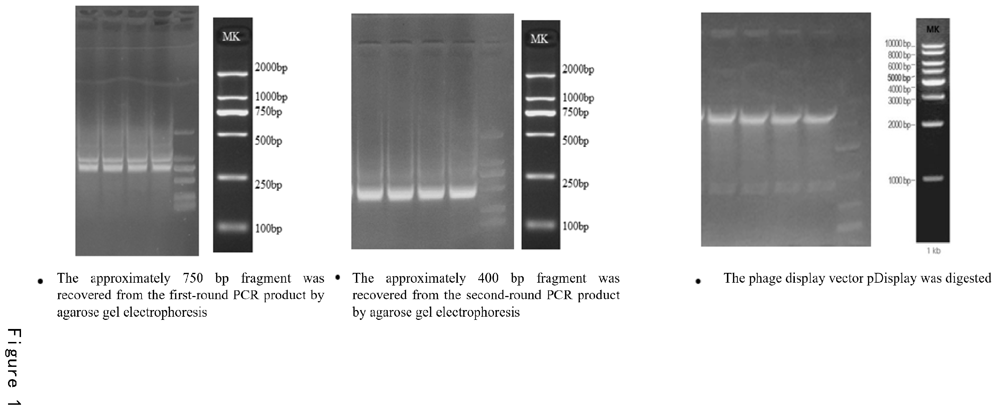

The results of the agarose gel electrophoresis of the PCR products are shown in Figure 1. In the first-round PCR, bands of approximately 1000 bp and 750 bp were obtained, and the 750 bp fragment was recovered as the template for the second-round PCR. In the second-round PCR, a band of approximately 400 bp, corresponding to the VHH fragment, was obtained. The pDisplay vector and the above-obtained VHH PCR gel-purified products were digested with SfiI, respectively. The digested pDisplay vector and VHH fragments were ligated using T4 ligase, and after overnight ligation at 16 °C, the ligation products were transformed into *E. coli*.

### 1.4 Construction of Phage Display Library

### 1. Construction of single-domain antibody phage display vector

The pDisplay vector and the above-obtained VHH PCR gel-purified products were digested with SfiI, respectively. The digestion systems are shown in Table 9 and Table 10:

**Table 9 Digestion system**

| Component | Amount |
|---|---|
| pDisplay | 140 µg |
| 10× CutSmart^{®} Buffer | 200 µL |
| SfiI | 1200 U (60 µL) |
| Sterile water | Bring to 2000 µL |

The digestion system shown in Table 9 was aliquoted into 100 µL/tube and placed in a PCR instrument for overnight digestion at 50 °C.

**Table 10 Digestion system**

| Component | Amount |
|---|---|
| Second-round PCR purified product | 10 µg |
| 10× CutSmart^{®} Buffer | 20 µL |
| SfiI | 360 U (18 µL) |
| Sterile water | Bring to 200 µL |

The digestion system shown in Table 10 was aliquoted into 100 µL/tube and placed in a PCR instrument for overnight digestion at 50 °C. The pDisplay vector fragments were separated using 1% agarose gel electrophoresis, and the 5000 bp vector fragment was excised and recovered by gel extraction. Meanwhile, the PCR-digested products were purified using a DNA fragment recovery kit, and the concentration was measured with a NanoDrop. The digested pDisplay vector and VHH fragments were ligated using T4 ligase, and the ligation was performed overnight at 16 °C.

### 2. Electroporation of phage ligation products into E. coli

Electroporation cuvettes, ligation products, and electrocompetent cells were pre-chilled on ice. The pre-chilled library ligation products were added to the electrocompetent cells and placed on ice for 1 min. To each cuvette, 300 µL of the DNA/competent cell mixture was added, and the cuvettes were placed on ice. Electroporation was performed at 2500 V for 5 ms. Immediately after electroporation, SOC medium pre-warmed to room temperature was added to resuspend the cells, and the cells were cultured in a shaking incubator at 37 °C for 1 hour. A 15 mL aliquot of the culture was directly used for phage rescue, while the remaining 5 mL of the electroporation product was mixed with an equal volume of 50% glycerol and stored at -80 °C after uniform mixing. In addition, 20 µL of the culture was taken and diluted by adding 980 µL of 2YT medium. Then, 100 µL of the diluted product was further diluted by adding 900 µL of 2YT medium, and 50 µL was spread evenly onto LB plates containing ampicillin and cultured overnight at 37 °C. The next day, the plates were removed, and the number of colonies generated per ligation was counted to calculate the library size. Meanwhile, 20 single colonies from the plates were picked and inoculated into 2YT medium containing ampicillin, and cultured with shaking at 37 °C for approximately 6-8 hours. The cultures were then sent for sequencing (using the universal primer M13R). Twenty randomly selected single colonies were sequenced to analyze the diversity of the constructed phage display library.

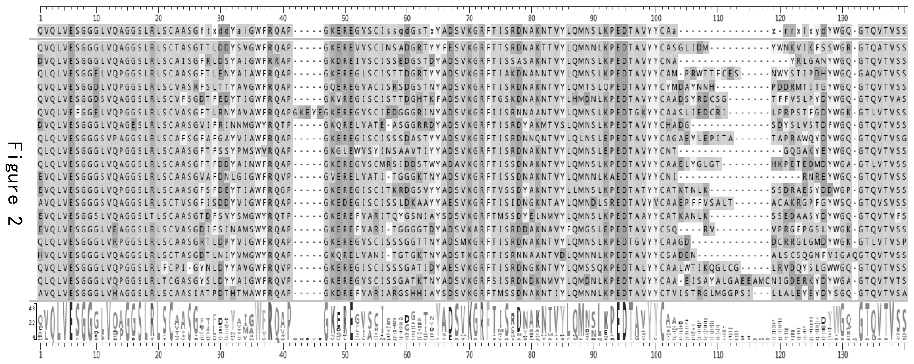

The results are shown in Figure 2. According to the alignment of the sequencing results, the empty vector rate and antibody repetition rate of the phage display library were no more than 5%, and the library size in *E. coli* was 1 × 10⁹.

### 1.5 Phage Library Preparation and Phage Precipitation

The electroporated transformation products were diluted with 2YT medium and adjusted to an OD₆₀₀ of approximately 0.2. Ampicillin was added to a final concentration of 100 µg/mL, and the culture was incubated in a constant-temperature shaker at 37 °C, 225 rpm, until the OD₆₀₀ reached 0.5. M13KO7 helper phage was then added, and after thorough mixing, the culture was incubated statically at 37 °C for 30 min, followed by incubation at 37 °C, 225 rpm for 1 h. The volume of M13KO7 added was calculated as: M13KO7 volume = 10 × culture volume × OD₆₀₀ × 5 × 10⁸ / M13KO7 titer. The bacterial culture was centrifuged at 6000 rpm for 10 min, and the pellet was resuspended in 2YT-AK medium and cultured overnight at 25 °C, 200 rpm. The bacterial culture was centrifuged at 10,000 rpm for 15 min. The supernatant was transferred to a new centrifuge tube, and 1/5 volume of PEG/NaCl was added, mixed thoroughly, and placed at 4 °C for 2 h. The precipitated phage supernatant was centrifuged at 10,000 rpm, 4 °C for 30 min. The supernatant was discarded, and the phage pellet in each 50 mL centrifuge tube was resuspended in 1 mL of sterile PBS. The resuspended phage was transferred to 1.5 mL EP tubes and centrifuged at 12,000 g, 4 °C for 5 min. The supernatant was transferred to new 1.5 mL EP tubes, and 250 µL of PEG/NaCl was added to each tube, mixed thoroughly, and incubated at 4 °C for 10 min. After centrifugation at 12,000 g for 10 min, the supernatant was discarded, and the pellet was resuspended in 1 mL of PBS, followed by centrifugation at 12,000 g for 5 min. The supernatant was transferred to new 1.5 mL EP tubes, and the centrifugation was repeated, yielding the original phage library. For titer determination, 10 µL of the precipitated phage was added to 90 µL of 2YT medium (designated as 10⁻¹), and serial 10-fold dilutions were performed up to 10⁻⁹. From the 10⁻⁷, 10⁻⁸, and 10⁻⁹ dilutions, 20 µL of each diluted sample was added to 200 µL of ER2738 cells (prepared at OD₆₀₀ = 0.5). After mixing, the samples were incubated statically in a 37 °C water bath for 10 min, and 100 µL of each was spread onto LB-AMP solid agar plates and cultured overnight at 37 °C. The next day, plaques were counted to determine the titer. The titer was calculated as follows: plates with 30-300 plaques were selected, the average of two plates was taken, and the plaque number was multiplied by the dilution factor and then by 100.

### 1.6 Panning of the Phage Display Library

Panning was performed using the recombinant protein. The phage display library was incubated with the recombinant protein, and the recombinant phages bound to the target antigen were eluted with TEA and amplified. After 3-4 rounds of panning, single clones were selected for sequencing.

Approximately 6 × 10¹¹ Pfu of the phage library (150 µL) was diluted to approximately 1 mL with 1% PBSA and added to the target protein-coated ELISA plate, followed by incubation at 4 °C for 1 h. Bound phages were eluted with 600 µL of 1× TEA for 10 min, and the eluted product was transferred to pre-blocked EP tubes and neutralized by adding 300 µL of Tris-HCl. For output titer determination, 10 µL of the output product was added to 90 µL of 2YT medium (designated as 100), and serial 10-fold dilutions were performed up to 10⁻². From the 101, 100, 10⁻¹, and 10⁻² dilutions, 20 µL of each diluted sample was added to 200 µL of ER2738 cells (prepared at OD600 = 0.5). For the 101 dilution, 20 µL of the undiluted output product was directly added to ER2738 cells. After mixing, the samples were incubated statically in a 37 °C water bath for 10 min, and 100 µL of each was spread onto LB-AMP solid agar plates and cultured overnight at 37 °C. The next day, plaques were counted to determine the titer. The titer was calculated as follows: plates with 30-300 plaques were selected, the average of two plates was taken, and the plaque number was multiplied by the dilution factor and then by the elution volume.

The target recombinant antigen protein was co-incubated with the single-domain antibody phage display library to enrich for phages specifically binding to the target antigen, through four rounds of enrichment. The input and output of each round were recorded, and the enrichment factor for each round was calculated. The results are shown in Table 11.

**Table 11 Enrichment results for each round**

| Round | Input (PFU) | Output(PFU) | Enrichment factor |
|---|---|---|---|
| 1 | 1.17E+13 | 3.96E+05 | 2.95E+07 |
| 2 | 4.80E+12 | 1.24E+06 | 3.87E+06 |
| 3 | 2.64E+12 | 5.40E+06 | 4.89E+05 |
| 4 | 4.82E+12 | 1.35E+06 | 3.57E+06 |

### 1.7 Phage ELISA

Single colonies from the third-round enriched products were selected for phage ELISA detection. The experimental procedure was as follows:
2YT-Amp medium (500 µL per well) was aliquoted into a 96-well deep-well plate. Single colonies from the third-round output plate were picked and cultured at 37 °C, 225 rpm until the OD₆₀₀ reached 0.5. Wells E12 and F12 were left without colony inoculation (only medium added) as blank controls. Wells G12 and H12 were also left without colony inoculation and were used to detect antigen binding to a positive antibody as positive controls. Meanwhile, ELISA plates were coated with antigen at a concentration of 1 µg/mL, 100 µL/well, at 37 °C for 2 h. Another 96-well deep-well plate was prepared with 500 µL per well of 2YT-A medium, and 10 µL of the bacterial culture at OD₆₀₀ = 0.5 was transferred to the corresponding wells of the new plate using a multichannel pipette. The plate was incubated overnight at 37 °C, 225 rpm for sequencing. To the bacterial culture at OD₆₀₀ = 0.5, M13KO7 helper phage was added, mixed thoroughly, and incubated statically at 37 °C for 15 min. The volume of M13KO7 added was calculated as: M13KO7 volume = 10 × culture volume × OD₆₀₀ × 5 × 10⁸ / M13KO7 titer. After infection, the plate was incubated in a shaker at 37 °C, 225 rpm for 45 min. The bacterial culture was then centrifuged at 4000 rpm for 10 min, the supernatant was discarded, and the pellet was resuspended in 2YT-AK medium (800 µL per well) and cultured overnight in a shaker at 30 °C, 210 rpm. Simultaneously, the antigen coating solution was discarded from the ELISA plate, and after three washes with PBST, the plate was blocked with 3% MPBS (250 µL/well) at 4 °C overnight. An additional blank plate was blocked as a BLANK control. On the next day, the 96-well deep-well plate was centrifuged at 4000 rpm for 10 min. The blocking buffer was discarded from the ELISA plate, and the plate was washed four times with 200 µL of PBST. PBST (50 µL) was added to each well, followed by 50 µL of the centrifuged phage supernatant, and the plate was incubated at 4 °C for 1 h. The supernatant was discarded, and the plate was washed five times with PBST. HRP-conjugated anti-M13 secondary antibody (diluted in PBST) was added at 100 µL/well and incubated at 4 °C for 45 min. After the secondary antibody was washed away with five PBST washes, TMB was developed at room temperature for 10 min, stopped with hydrochloric acid, and the absorbance was read. Clones with an S/N ratio greater than 6 were selected, and the corresponding glycerol stocks were sent for sequencing. The sequencing results were analyzed, and different antibody sequences were selected for construction of eukaryotic expression vectors.

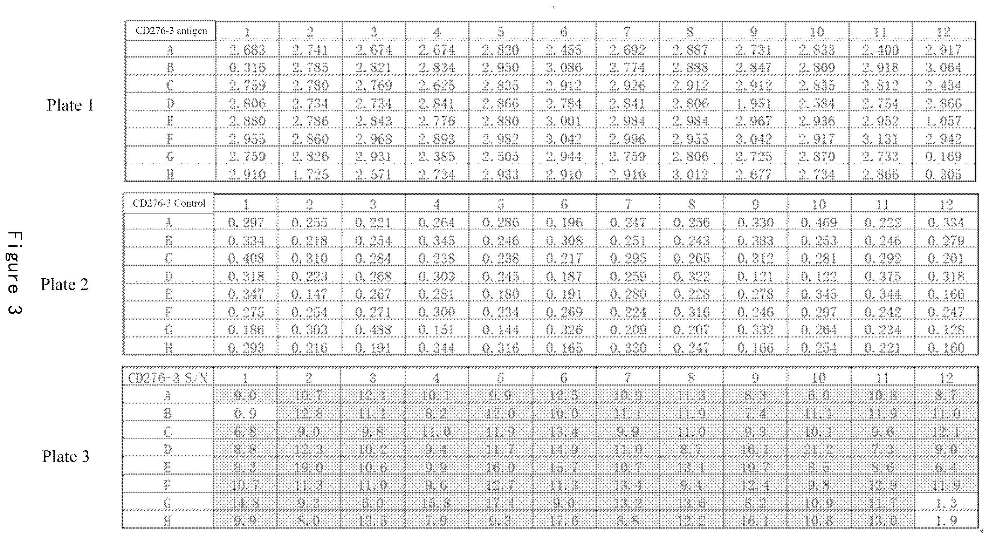

The results are shown in Figure 3. Plate 1 shows the OD450 values for monoclonal detection with the target antigen protein coated in the 96-well plate; Plate 2 shows the OD450 values for monoclonal detection with BSA as a control antigen protein coated in the 96-well plate; the S/N values in Plate 3 were calculated as the values shown in Plate 1 divided by the values of the corresponding wells in Plate 2. Clones with S/N values > 4 were selected for sequencing.

The sequence information of the candidate antibodies is shown below (the underlined regions indicate the CDR regions).
>1-B1 (SEQ ID NO: 1) 
AVQLVESGGGSVQPGGSLRLSCAASGFTFDDYYIGWFRQAVGKEREGVSWI
HSRSGSRYYADSVKGRFTVSSDNAKNTVYLQMNSLKPEDTAVYYCAVSRRGTSW
RLRVTDFGSWGQGTQVTVSS
>8-E5 (SEQ ID NO:2) 
AVQLVDSGGDSVQPGGSLRLSCSVSGMIISFNIMGWYRQAPGKQRELVGYIF
NNGNTYYSDSVKGRFTVSRDNARNTVNLQMNSLKPDDTGVYYCNTVAPLGSW
GQGTQVTVSS
>1-E3 (SEQ ID NO:3) 
AVQLVESGGGLVQPGGSLRLSCAASGIRFSINHMGWYRQAPGKQREFVASLP
NGGRTIYVGSVKGRFTISRDNAKNTVYLQMNRLEPEDTAVYYCNTVPPDNYWG
QGTQVTVSS
>1-A2 (SEQ ID NO: 4) 
QVQLVESGGGLVQSGGSLRLSCAASGRTFSRYTMGWFRQAPGKEREFVAAM
SWLGPTYYADSVRGRFTISRDNTKNTVYLQMNSLKPEDTAVYYCAADRAPAVVS
PEVDYWGQGTQVTVSS
>4-B11 (SEQ ID NO: 5)

EVQLVESGGGLVQAGDSLTLSCAASGRTFSRFIMAWFRQAPGKEREFVGRTN
RWSGDSTYVADSVKGRFTISRDNAKNIVYLQMNSLKPEDTAIYYCAARAATMGN

YDYWGQGTQVTVSS

**Table 12. Antibody CDR sequences**

| **Antibody name** | **CDR1** | **CDR2** | **CDR3** | **The amino acid sequence identifier of the VHH chain** |
|---|---|---|---|---|
| 1-B1 | GFTFDDYY | IHSRSGSR | AVSRRGTSWRLRVTDF GS | 1 |
| 8-E5 | GMIISFNI | IFNNGNT | NTVAPLGS | 2 |
| 1-E3 | GIRFSINH | LPNGGRT | NTVPPDNY | 3 |
| 1-A2 | GRTFSRYT | MSWLGPT | AADRAPAVVSPEVDY | 4 |
| 4-B11 | GRTFSRFI | TNRWSGDST | AARAATMGNYDY | 5 |

| | | | | |
|---|---|---|---|---|
| Note: The numerical values in the table represent sequence identifiers, i.e., "1" indicates "SEQ ID NO: 1." | | | | |

### Example 2: Evaluation and Characterization of Single-Domain Antibodies Against CD276

### 2.1 Preparation of Candidate Anti-CD276 Nanobodies

L0VTransm transfection reagent and antibody expression vectors were taken out from the refrigerator, thawed at room temperature, and mixed thoroughly by pipetting up and down. PBS buffer was taken out and warmed to room temperature. 2 mL of PBS was added to one well of a 6-well plate, and 50 µg of antibody eukaryotic expression vector was added respectively, mixed thoroughly by pipetting up and down, then 150 µL of LVTransm was added, immediately mixed thoroughly by pipetting up and down, and incubated at room temperature for 10 minutes. The above DNA/LVTransm complexes were added to 50 mL of 293F cells, gently shaken to mix thoroughly. The cells were placed in a 37 °C, 5% CO₂ incubator and cultured at 130 RPM. After continuous culture for 5-7 days, the culture supernatant was collected by centrifugation, filtered through a 0.45 µm filter membrane, and the filtrate was transferred to a sterile centrifuge tube. The antibodies were purified using a Protein A affinity column.

### 2.2 Flow Cytometry Detection of Binding of Candidate Antibodies to Target Protein

CHO and CHO-CD276 cell lines were recovered from liquid nitrogen, and the cell status was adjusted to logarithmic growth phase using complete medium. The cells were divided into several aliquots, with 3 × 10⁵ cells per aliquot. The expressed antibodies were incubated with the target cells respectively, mixed thoroughly, and incubated at room temperature for 1 hour. After centrifugation at 800×g at room temperature for 5 minutes, the supernatant containing the antibodies was removed, and the cells were washed 3 times with PBS. Goat anti-Human IgG (H+L) Cross-Adsorbed Secondary Antibody, Alexa Fluor 488 (diluted 1:1000), was added, mixed thoroughly, and incubated at room temperature in the dark for 30 minutes. After centrifugation at 800×g at room temperature for 5 minutes, the supernatant containing the secondary antibody was removed, and the cells were washed 3 times with PBS. The cells were resuspended in 500 µL of PBS for flow cytometry analysis.

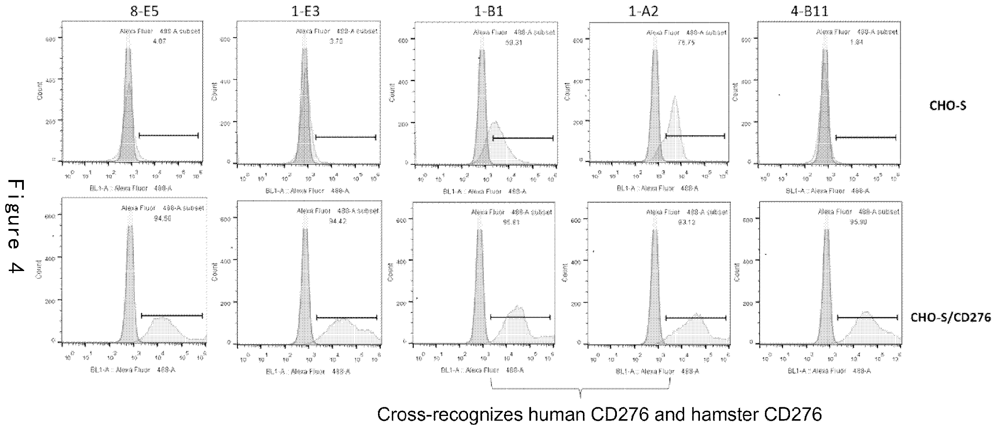

The results are shown in Figure 4. According to the flow cytometry results, all five candidate antibodies 1-B1, 8-E5, 1-E3, 1-A2, and 4-B11 were able to bind effectively to CD276; among them, 1-B1 and 1-A2 were cross-binding antibodies against human and hamster CD276.

### 2.3 Internalization Assay of Candidate Antibodies

SKOV3 cells in logarithmic growth phase were collected and divided into groups at 5 × 10⁵ cells per well. Purified antibodies (10 µg/mL, 200 µL/well) were added and incubated at 4 °C for 30 min. After centrifugation at 800×g at 4 °C for 5 min, the supernatant containing the antibodies was removed, and the cells were washed 3 times with pre-cooled PBS. The cells were evenly divided into two portions and incubated at 4 °C and 37 °C for 240 min, respectively. After incubation, ice-cold PBS was immediately added to stop the internalization, followed by centrifugation at 800×g at 4 °C for 5 min, and the cells were resuspended in 100 µL of pre-cooled PBS. Goat anti-Human IgG (H+L) Cross-Adsorbed Secondary Antibody, Alexa Fluor 488 (diluted 1:1000), was immediately added and incubated at 4 °C for 30 min. After centrifugation at 800×g at 4 °C for 5 min, the supernatant containing the antibodies was removed, and the cells were washed 3 times with pre-cooled PBS. The cells were resuspended in 200 µL of pre-cooled PBS and immediately analyzed by flow cytometry.

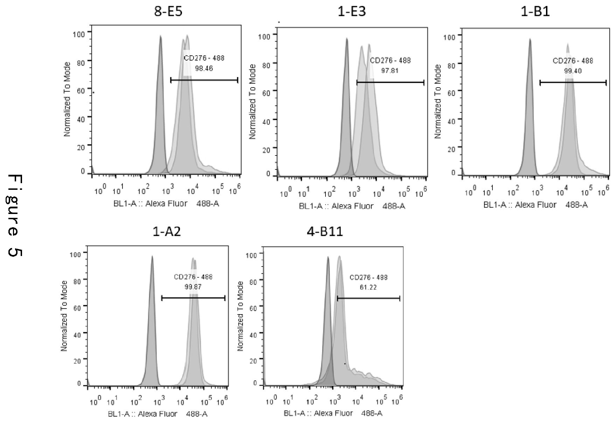

The results are shown in Figure 5. According to the flow cytometry internalization results, all five candidate antibodies exhibited internalization activity in SKOV3 cells, among which 8-E5, 1-E3, and 1-B1 showed high internalization activity.

### 2.4 ADC Cytotoxicity Assay of Candidate Antibodies

SKOV3 target cells (3.0 × 10⁵) in logarithmic growth phase were transferred to a 15 mL centrifuge tube, mixed thoroughly, and centrifuged at 500×g for 5 min (referencing the amount for one 96-well plate). After centrifugation, the supernatant was removed, and the cells were resuspended in 10 mL of DMEM complete medium. After thorough mixing, 100 µL of the cell suspension was added to each well of a 96-well plate. Surrounding unused wells were filled with 200 µL of sterile water. After seeding, the plate was transferred to a 37 °C, 5% CO₂ cell culture incubator and cultured for 24 h. After overnight culture, the 96-well plate was removed, and 100 µL of serially diluted ADC antibodies were added to the corresponding wells at final concentrations of 10, 2, 0.4, 0.08, 0.016, 0.0032, 0.00064, 0.000128, 0.0000256, and 0 µg/mL. The co-cultured 96-well plate was transferred to a cell culture incubator and cultured at 37 °C, 5% CO₂ for an additional 96 h. After culture, the 96-well plate was removed, and 20 µL of CCK-8 detection reagent was added to each well, avoiding bubble formation during addition. The plate was incubated at room temperature in the dark for 1, 2, 3, and 4 h, and the OD450 absorbance value was measured using a Tecan M1000 multifunctional microplate reader.

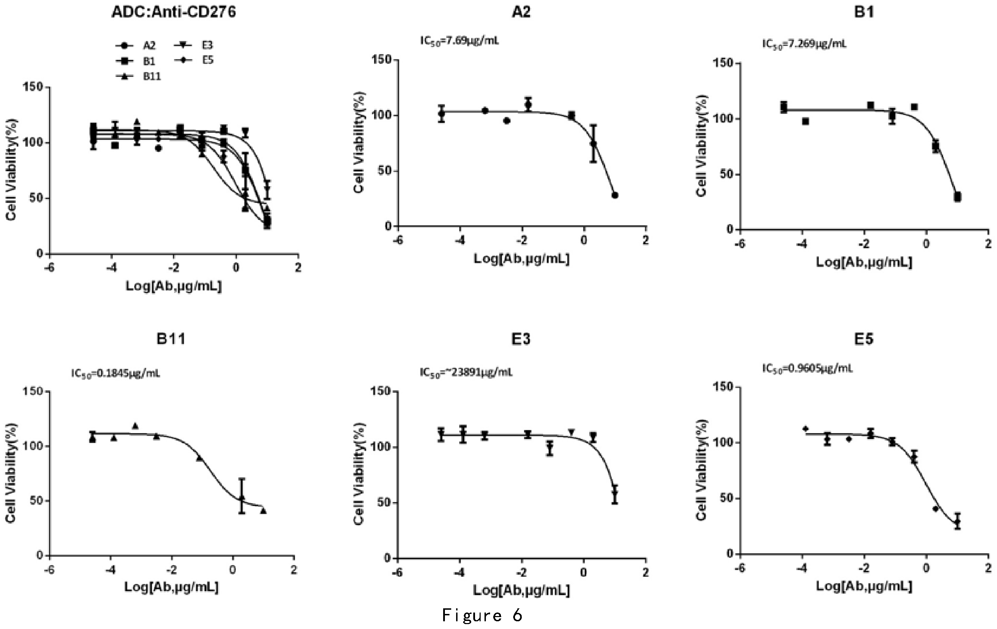

The results are shown in Figure 6 and Table 13. The conjugates of the candidate antibodies with the drug all exhibited cytotoxic activity, among which 4-B11 and 8-E5 showed particularly significant cytotoxic activity.

**Table 13 IC₅₀ values of candidate antibody ADCs**

| ADC-CD276 | IC50(µg/mL) |
|---|---|
| 1-A2 | 7.69 |
| 1-B1 | 7.269 |
| 4-B11 | 0.1845 |
| 1-E3 | NA |
| 8-E5 | 0.9605 |

### 2.5 Antibody Affinity Detection

Affinity detection was performed on 8-E5, 1-E3, and 1-B1. AHC sensors were used to immobilize the candidate antibodies, with an immobilization time of 40 s. The buffer was PBST (PBS + 0.02% Tween 20), and CD276-His protein was diluted to 200, 100, 50, 25, 12.5, and 0 nM. Affinity detection conditions: equilibration for 60 s, association for 180 s, dissociation for 180 s, and the detection temperature was 25 °C.

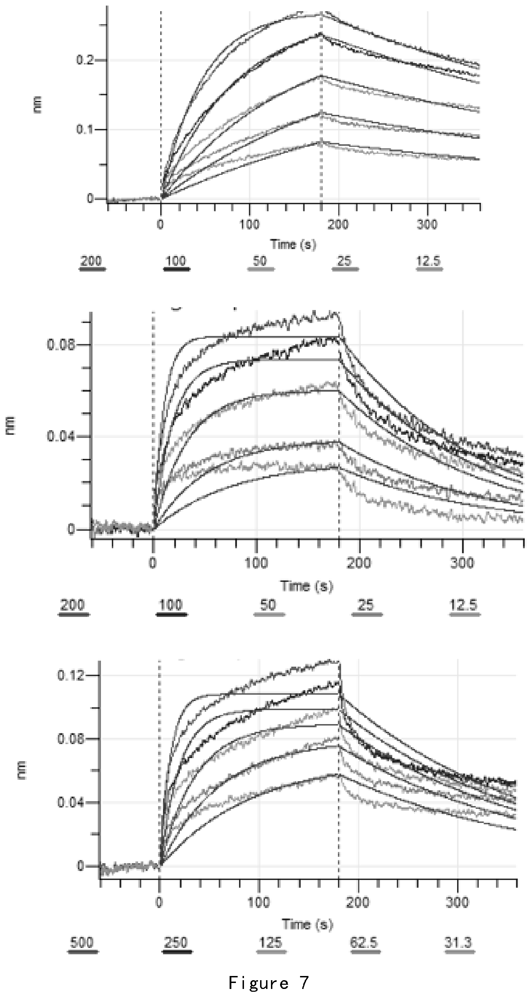

The results are shown in Figure 7 and Table 14. The antibodies 8-E5, 1-E3, and 1-B1 of the present invention all exhibited high affinity.

**Table 14 Antibody affinity detection results**

| Antibody Name | ka M⁻¹s⁻¹ | kd s⁻¹ | KD M |
|---|---|---|---|
| 1-B1 | 1.006x10⁵ | 1.953x10⁻³ | 1.941x10⁻⁸ |
| 1-E3 | 4.719x10⁵ | 7.334x10⁻³ | 1.554x10⁻⁸ |
| 8-E5 | 1.853x10⁵ | 5.148x10⁻³ | 2.779x10⁻⁸ |

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. An anti-CD276 nanobody, wherein the anti-CD276 nanobody has one or more complementarity-determining regions (CDRs) selected from the group consisting of:
(1) CDR1 as set forth in SEQ ID NO: 6, CDR2 as set forth in SEQ ID NO: 7, and CDR3 as set forth in SEQ ID NO: 8;
(2) CDR1 as set forth in SEQ ID NO: 9, CDR2 as set forth in SEQ ID NO: 10, and CDR3 as set forth in SEQ ID NO: 11;
(3) CDR1 as set forth in SEQ ID NO: 12, CDR2 as set forth in SEQ ID NO: 13, and CDR3 as set forth in SEQ ID NO: 14;
(4) CDR1 as set forth in SEQ ID NO: 15, CDR2 as set forth in SEQ ID NO: 16, and CDR3 as set forth in SEQ ID NO: 17;
(5) CDR1 as set forth in SEQ ID NO: 18, CDR2 as set forth in SEQ ID NO: 19, and CDR3 as set forth in SEQ ID NO: 20.

2. The anti-CD276 nanobody of claim 1, wherein the CDR1, CDR2 and CDR3 are separated in sequence by framework regions FR1, FR2, FR3 and FR4 of a VHH chain, respectively.

3. The anti-CD276 nanobody of claim 1, wherein the amino acid sequence of the VHH chain of the anti-CD276 nanobody is as shown in any one of SEQ ID NOs: 1-5.

4. The anti-CD276 nanobody of claim 1, wherein the anti-CD276 nanobody compries humanized antibodies, animal-derived antibodies, and chimeric antibodies.

5. An anti-CD276 multivalent antibody or antibody against multiple epitopes, wherein the multivalent antibody or antibody against multiple epitopes comprises at least one antibody element against a CD276 epitope, and the antibody element is the anti-CD276 nanobody of claim 1.

6. A recombinant protein, wherein the recombinant protein comprises:
(i) the anti-CD276 nanobody of claim 1, or the anti-CD276 multivalent antibody or antibody against multiple epitopes of claim 5; and
(ii) an optional tag sequence that facilitates expression and/or purification.

7. An antibody-drug conjugate, wherein the antibody-drug conjugate comprises:
(a) the nanobody of claim 1, the multivalent antibody or antibody against multiple epitopes of claim 5, or the recombinant protein of claim 6; and
(b) a conjugated moiety conjugated to the antibody moiety, wherein the conjugated moiety is selected from the group consisting of: detectable labels, drugs, toxins, cytokines, radionuclides, enzymes, and combinations thereof.

8. A chimeric antigen receptor (CAR), wherein the CAR comprises an extracellular domain that comprises the anti-CD276 nanobody of claim 1, or the anti-CD276 multivalent antibody or antibody against multiple epitopes of claim 5.

9. A polynucleotide, wherein the polynucleotide encodes a protein selected from the group consisting of: the nanobody of claim 1, the multivalent antibody or antibody against multiple epitopes of claim 5, the recombinant protein of claim 6, and the CAR of claim 8.

10. An expression vector, wherein the expression vector comprises the polynucleotide of claim 9.

11. A host cell, wherein the host cell comprises the expression vector of claim 10, or has the polynucleotide of claim 9 integrated into its genome, or expresses the nanobody of claim 1, the multivalent antibody or antibody against multiple epitopes of claim 5, the recombinant protein of claim 6, or the CAR of claim 8.

12. A pharmaceutical composition, comprising:
(i) the nanobody of claim 1, the multivalent antibody or antibody against multiple epitopes of claim 5, the recombinant protein of claim 6, the antibody-drug conjugate of claim 7, or the host cell of claim 11; and
(ii) a pharmaceutically acceptable carrier.

13. Use of the nanobody of claim 1, the multivalent antibody or antibody against multiple epitopes claim 5, the recombinant protein of claim 6, the antibody-drug conjugate of claim 7, or the immune cell expressing the CAR of claim 8 in the preparation of a medicament for preventing and/or treating cancers or diseases associated with CD276.

14. The use of claim 13, wherein the cancers or diseases is selected from the group consisting of: leukemia, gastric cancer, breast cancer, non-small cell lung cancer, ovarian cancer, osteosarcoma, neuroblastoma, pancreatic cancer, breast cancer, and combinations thereof.

15. A method for treating a disease, wherein the method comprises administering to a subject in need of treatment: the nanobody of claim 1, the multivalent antibody or antibody against multiple epitopes claim 5, the recombinant protein of claim 6, the antibody-drug conjugate of claim 7, or the immune cell expressing the CAR of claim 8.
